Europäisches Patentamt

European Patent Office

Office européen des brevets

(19)

(11) Publication number: **0 332 416**
A2

(12) **EUROPEAN PATENT APPLICATION**

(21) Application number: **89302296.2**

(22) Date of filing: **08.03.89**

(51) Int. Cl.⁴: **C 12 N 15/00**
C 12 N 5/00, C 12 P 21/00

(30) Priority: **11.03.88 US 167055**

(43) Date of publication of application:
**13.09.89 Bulletin 89/37**

(84) Designated Contracting States:
**AT BE CH DE ES FR GB GR IT LI LU NL SE**

(71) Applicant: **DANA-FARBER CANCER INSTITUTE, INC.**
**44 Binney Street**
**Boston Massachusetts MA 02115 (US)**

(72) Inventor: **Livingston, David M.**
**236 Walnut Street**
**Brookline Massachusetts 02146 (US)**

**Figge, James J.**
**650 Huntington Avenue No. 17H**
**Boston Massachusetts 02115 (US)**

**Wright, Christopher I.**
**39 Skehan Street No. 3**
**Somerville Massachusetts 02143 (US)**

**Decaprio, James A.**
**Five Putney Road**
**Wellesley Massachusetts 02180 (US)**

**Roberts, Thomas M.**
**13 Berkley Street**
**Cambridge Massachusetts 02138 (US)**

**Brown, Myles A.**
**25 Englewood Avenue No. 3**
**Brookline Massachusetts 02146 (US)**

(74) Representative: **Field, Roger Norton et al**
**Brewer & Son Quality House Quality Court Chancery Lane**
**London WC2A 1HT (GB)**

(54) Regulation of expression in mammalian cells.

(57) Synthesis of a desired protein in a mammalian cell is regulated by transfecting the cell with a eukaryotic promoter coupled to a DNA sequence encoding a prokaryotic repressor, transfecting the cell with a prokaryotic operator linked to a DNA sequence encoding the desired protein and a promoter driving the transcription of the protein-encoding DNA sequence, the operator and the repressor-encoding DNA sequence being functional in the cell, and culturing the cell in the presence or absence of a substance that binds the repressor.

EP 0 332 416 A2

**Description**

## REGULATION OF EXPRESSION IN MAMMALIAN CELLS

This invention relates to gene expression in mammalian cells.

Regulation of expression of chromosomal genes in cells can be accomplished via the action of certain proteins which bind to specific DNA sequences and either activate or repress expression of specific genes. Eukaryotic cells can respond to environmental stimuli by the action of certain DNA binding proteins which also interact with small effector molecules. For example, the glucocorticoid receptor protein can bind to both a specific chromosomal enhancer sequence termed the glucocorticoid response element ("GRE") and to glucocorticoid hormones such as dexamethasone. In the presence of an appropriate extracellular concentration of dexamethasone, the hormone-glucocorticoid receptor complex can activate the transcription of genes which are linked to a GRE. Moreover, the region of the protein responsible for specific DNA binding is essential for transcriptional activation, J. Carlstedt-Duke et al., 1987, Proc. Natl. Acad. Sci. USA, vol. 84, pp. 4437-40; V.L. Chandler et al., 1983, Cell, vol. 33, pp. 489-99; S.M Hollenberg et al., 1985, Nature, vol. 318, pp. 635-41; S.M. Hollenberg et al., 1987, Cell, vol. 49, pp. 39-46; N.E. Hynes et al., 1981, Proc. Natl. Acad. Sci. USA, vol. 78, pp. 2038-42; F. Lee et al., 1981, Nature, vol. 294, pp. 228-32; R. Miesfeld et al., 1987, Science, vol. 236, pp. 423-27; S. Rusconi et al., 1987, EMBO J., vol. 6, pp. 1309-15.

Several inducible promoter systems have been used for achieving regulated expression of genes introduced into eukaryotic cells by gene transfer, including promoters inducible by heat shock, C. Wu, 1984, Nature, vol. 309, pp. 229-34; the metal ion-inducible metallothionein promoter, K.E. Mayo et al., 1982, Cell, vol. 29, pp. 99-108; the glucocorticoid-inducible mouse mammary tumor virus long terminal repeat, reviewed by G.M. Ringold, 1983, Curr. Topics Microbiol. Immunol., vol. 106, pp. 79-103; and poly(IC)-inducible interferon promoters, S. Goodbourn et al., 1985, Cell, vol. 41, pp. 509-20, J. Ryals et al., 1985, Cell, vol. 41, pp. 497-507.

Prokaryotes also can regulate expression of their chromosomal genes in response to certain environmental stimuli. For example, the bacterium E. coli can synthesize enzymes (e.g., β-galactosidase) which allow it to utilize lactose as an energy source. This induced response is mediated by a specific DNA binding protein, the lac repressor, J.H. Miller et al., 1980, The Operon, Cold Spring Harbor, New York. This protein, composed of four identical 38.6 kd subunits, K. Beyreuther, 1980, in J.H. Miller et al., supra, pp. 123-54, can bind both to allolactose (or an analogue) and to a cognate lac operator sequence which is normally embedded within the bacterial chromosome. When bound to the operator, the repressor prevents transcription of the lac operon. However, when bound to an inducer such as isopropyl β-D-thiogalactoside ("IPTG"), the repressor binds with 300-fold lower affinity to the operator, M.D. Barkley et al., 1980, in J.H. Miller et al., supra, pp. 177-220, thereby activating transcription of the operon.

R. Brent et al., 1984, Nature, vol. 312, pp. 612-15, describe binding the E. coli lexA repressor protein to its operator in yeast and blocking thereby of transcription of a gene on a chromosome of yeast.

M. C.-T. Hu et al., 1987, Cell, vol. 48, pp. 555-66, describes regulation by the E. coli lac repressor protein of expression in mouse cells of a gene that is not integrated into the host cell chromosome. Hu et al. cotransfected mouse LTK-cells with a eukaryotic expression vector containing a lacI gene and with a plasmid containing a tk gene, selected stable transfectants by growth in HAT medium, and screened clonal isolates for stably expressed lacI mRNA and repressor protein, to obtain a cell line in which functional lac repressor was expressed. Hu et al. then transfected the repressor-positive cells with plasmids containing a MSV-CAT fusion gene and a SV40 early promoter region, into which they had inserted a lac operator sequence. In these repressor-positive cells containing the unintegrated MSV-CAT fusion genes, CAT expression was repressed by the lac repressor in the absence of IPTG, and derepressed by induction with IPTG in the culture medium.

In general, the invention features, in one aspect, a mammalian cell containing a DNA sequence encoding a functional prokaryotic repressor, and containing a functional cognate operator integrated into a chromosome of the cell.

An operator and a repressor are "cognate", as that term is used herein, where the repressor specifically binds to the operator and thereby inhibits transcription of a gene linked to the operator.

A repressor and its cognate operator are "functional" in host cell, as that term is used herein, where the repressor is capable of preventing initiation in the host cell of transcription of a DNA sequence linked to the operator.

In preferred embodiments, the cell further contains, linked to the operator and integrated into a chromosome, a DNA sequence encoding a desired protein together with a promoter driving its transcription; the repressor comprises a lac repressor and the operator comprises a lac operator; the lac operator is situated within or downstream from the promoter and upstream from the DNA sequence encoding the desired protein; the DNA sequence encoding the lac repressor is integrated into a chromosome of the cell; the lac operator and the DNA sequence encoding the lac repressor are derived from E. coli; the promoter is derived from SV40; the lac repressor gene includes a DNA sequence derived from an E. coli lacI gene by modifying the initiation codon of the lacI gene from GTG to ATG; the lac operator includes a mutant E. coli lac operator DNA sequence; and the lac operator includes a synthetic DNA sequence comprising the nucleotide sequence 5'-ATTGTGAGCGCTCACAAT-3'.

In another aspect, the invention features a method for regulating synthesis of a desired protein in a mammalian cell, including transfecting the cell with a DNA sequence encoding a prokaryotic repressor coupled to a eukaryotic promoter, and with a prokaryotic operator linked to a DNA sequence encoding the desired protein together with a promoter driving its transcription, the operator and the linked DNA sequence being integrated into a chromosome of the cell, the operator and the DNA sequence encoding the repressor being functional in the cell; and culturing the cell in the presence of a substance that binds the repressor to provide synthesis of the desired protein, or culturing the cell in the absence of a substance that binds the repressor to inhibit synthesis of the desired protein.

The DNA sequence encoding the desired protein is linked to an operator, as that term is used herein, when the operator is located within the promoter that drives transcription of the DNA sequence encoding the desired protein, in a position where the operator is capable of being bound to repressor and when so bound inhibits transcription of the linked DNA sequence and where the operator does not interfere with the promoter function when the operator is not bound by repressor; or when the operator is located between the promoter that drives transcription of the DNA sequence and the DNA sequence itself.

It has been discovered that a prokaryotic repressor can recognize and bind its cognate operator sequence when the latter is integrated into a mammalian chromosome; where such a cognate operator sequence is linked to a gene which is stably integrated into a chromosome, binding of the repressor to the operator can substantially block expression of the gene. Moreover, the presence of a molecule that binds the repressor can lead to reactivation of the operator-linked gene. Binding of the lac repressor to the lac operator inhibits transcription from the promoter that drives transcription of the linked DNA sequence encoding the desired protein, and the lac repressor can be used to stringently regulate expression of a stably integrated gene in healthy mammalian cells. Stable repression of the gene can be reversed by the presence of allolactose or an analog of allolactose such as IPTG in the extracellular environment of the cells.

Transcription from the promoter of the DNA sequence driven by it is inhibited by binding of the lac repressor to the lac operator, and the DNA sequence encoding the desired protein can be derepressed by the presence in the cellular environment of a specific lac repressor binding substance, allolactose or an analog of allolactose, which, in turn, reduces the affinity of the repressor for the lac operator.

In another aspect, the invention features a mammalian organism including a mammalian cell containing a functional lac operator integrated into a chromosome, and containing a DNA sequence encoding a functional lac repressor.

In another aspect, the invention features a mammalian organism including a mammalian cell that includes a DNA sequence encoding a functional lac repressor.


Description of a Preferred Embodiment


Drawings

Fig. 1 is a restriction map of a plasmid containing a cloned lacI coding sequence with a modified initiation codon under control of a eukaryotic promoter element;

Fig. 2 is a restriction map of a plasmid containing a promoter/enhancer sequence, a lac operator sequence and a DNA sequence encoding a desired protein;

Fig. 3 is a restriction map showing a deduced configuration of the integration of pSVlacOCAT DNA into host chromosomal DNA.

M. Brown et al., 1987, Cell, vol. 49, pp. 603-12, describes regulation by the E. coli lac repressor protein of expression in monkey cells and in mouse cells of genes that are not integrated into the host cell chromosome. Brown et al. cotransfected monkey cells with a plasmid containing a lacI coding sequence driven by a CMV promoter and with a plasmid containing a CAT gene linked to a lac operator-containing SV40 early promoter-enhancer, and showed that expression of CAT from the lac operator-linked CAT gene on unintegrated templates was repressed by lac repressor protein, and that this repression was overcome in the presence of IPTG. Brown et al. also cotransfected NIH-3T3 mouse cells with a plasmid containing a lacI coding sequence driven by a mouse metallothionein-I promoter and with a plasmid containing a neomycin resistance gene, and selected a stable cell line in G418-supplemented media that stably produced a protein apparently identical to lac repressor. They then transfected these lacI⁺ cells with a plasmid containing a CAT gene linked to a lac operator-containing SV40 early promoter enhancer element and incubated transfectants with and without IPTG; CAT expression from lac operator-linked templates was repressed in these cells in the absence of IPTG, and derepressed in the presence of IPTG.


Structure

Transfected mammalian cells according to the invention can be made and the method of the invention can be practiced using standard techniques of nucleic acid manipulation. In the example that follows, presented by way of illustration, a mammalian cell line is made that uses a lac repressor-operator regulatory apparatus and IPTG as a derepressor for regulating expression of chloramphenicol acetyl transferase ("CAT").

A stable mammalian cell line capable of expressing lac repressor ("lacI⁺ cells") and containing an integrated DNA sequence encoding a desired protein ("desired DNA") whose promoter is linked to a lac operator can be made generally as follows. The cells are cotransfected with plasmids containing the lac repressor, the desired DNA containing a linked lac operator, and a selectable marker. Transfectants are selected by means of the selectable marker, and the transfectants are screened for expression of the desired protein in both the presence and the absence of an allolactose analog that binds lac repressor. Cell lines that express the desired protein in a regulated fashion, that is, in the presence of, but not in the absence of, the allolactose analog are then maintained in actively growing culture for an extended time, and cell lines that continue to present allolactose analog-inducible expression of the desired protein can then be shown to have integrated the lac operator-containing target gene and not to harbor free copies of this sequence by molecular hybridization methods demonstrating both an absence of free lac operator-target gene containing plasmids and incorporation of the this DNA sequence into a host cell chromosome.

Use

A prokaryotic repressor and, inserted into a promoter that is functional in mammalian cells, its cognate operator, can be introduced into any mammalian cell line and can be used to tightly regulate the expression of any desired DNA sequence whose transcription is driven by the operator-linked promoter. The expression of a desired gene in a culture of mammalian cells that has been so transfected according to the invention can be tightly regulated by varying the concentration in the culture medium of a specific inducer or derepressor having low toxicity to the cells.

Moreover, the expression of a desired gene in a living mammalian organism can be regulated by, for example, infecting by known techniques all of the cells of the organism or a portion of the cells of a tissue or organ of the organism with a suitable viral vector, such as, for example, a retrovirus, encoding a lac repressor and containing a lac operator inserted into the promoter driving expression of the desired gene. Plasmids containing functional lac repressor and a lac operator-linked desired gene could also be concurrently introduced into a germ cell or a zygote or a tissue primordium within an embryo by microinjection, for example, and the resulting organisms screened for IPTG-inducible expression of the desired gene.

Alternatively, separate transgenic strains of a given mammalian species carrying each of these genes can be mated, and progeny carrying both the lacI and the desired lac operator-linked gene can be identified and inbred so as to create a stable strain whose phenotype is lacI+ and lac operator-linked gene+.

Thus, for example, the porcine growth hormone in pigs or the bovine growth hormone in cattle can be regulated by feeding or injecting the transgenic animal with selected amounts of IPTG at selected points during the animal's development, for enhancing meat production without activating unhealthy growth of boney structures. Similarly, for example, insulin secretion can be regulated in type II human diabetics by introduced the lacI and a lac operator-containing human insulin gene, each driven by a suitable promoter, into an autologous cell which can be engrafted into the patient. Insulin expression can then be activated and repressed by withholding and administering IPTG or a suitable analog.

## Example

The following example, presented by way of illustration, describes the construction of stable, G418-resistant monkey cell lines having an IPTG-inducible CAT+ phenotype.

### Plasmids and Their Construction.

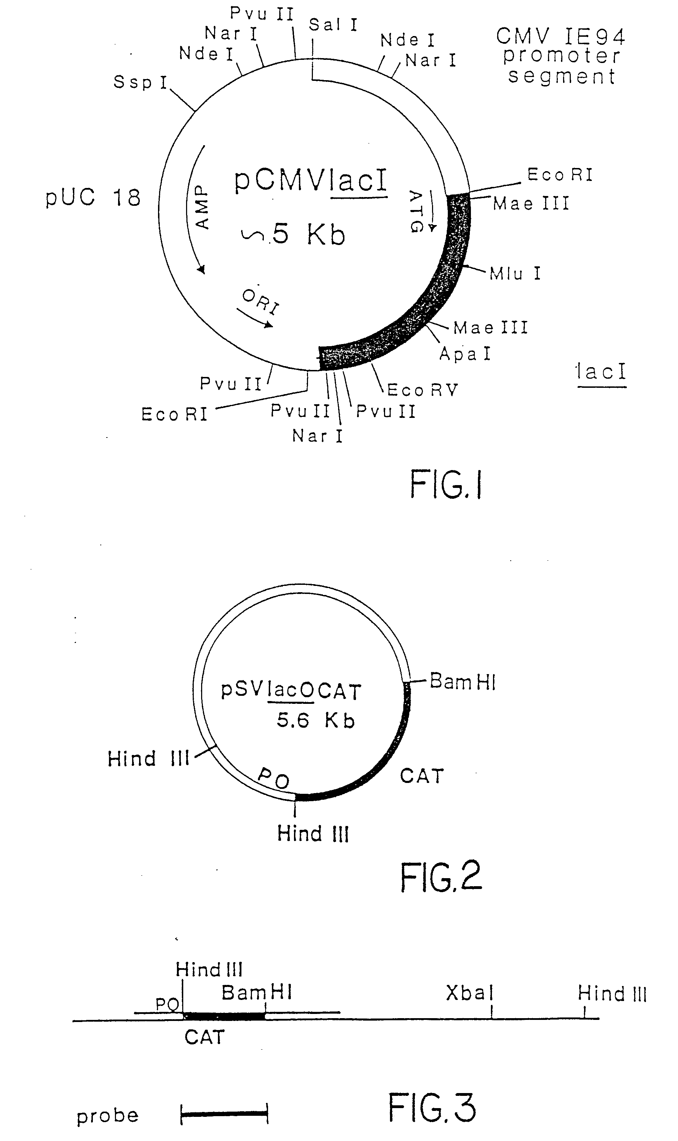

With reference to Fig. 1, there is shown a restriction map, prepared using standard methods of restriction cleavage, of plasmid pCMVlacI, which can be used for transfecting a mammalian cell so that the cell presents a lacI+ phenotype. Plasmid pCMVlacI, described in Brown et al , 1987, Cell, vol. 49, pp. 603-612, contains a cloned lacI coding sequence with a modified initiation codon (ATG) under control of a simian cytomegalovirus (Colburn) IE94 promoter element. Restriction endonuclease sites which have been experimentally verified are shown. The sites for SspI, SalI, MluI, ApaI, and EcoRV are unique. The MaeIII site in the mid portion of the lacI gene is contained within a unique BstEII site. The sites mapped within the lacI coding unit are in complete agreement with the knownlacI sequence, as described in Farabaugh, 1978, Nature, vol. 274, pp. 765-769. The entire lacI coding unit is flanked by two EcoRI sites. There is no site present for XbaI.

With reference now to Fig. 2, there is shown a restriction map of plasmid pSVlacOCAT, which can be used for transfecting a mammalian cell so that the cell presents a CAT+ phenotype. Plasmid pSVlacOCAT, described in Brown et al., 1987, Cell, vol. 49, pp. 603-612, contains an SV40 promoter/enhancer sequence ("P"), a mutant, symmetrical lac operator sequence and a chloramphenicol acetyl transferase ("CAT") coding sequence.

All cleavages and ligations were performed according to standard methods, as described, for example, in Maniatis et al., 1982, Molecular Cloning: A Laboratory Manual, Cold Spring Harbor, New York. Restriction endonucleases were purchased from Boehringer Mannheim and New England Biolabs. Plasmid pCMVlacI was constructed generally as outlined by Brown et al., 1987, Cell, vol. 49, pp. 603-612. The nucleotide sequence of the simian CMV (Colburn) IE94 promoter and enhancer region is described in Jeang et al., 1987, J. Virol., vol. 61. pp. 1559-1570.

Plasmid pSVlacOCAT was constructed generally as follows. An oligonucleotide was synthesized chemically containing 22 bases and having the structure:
5′-TCGAATTGTGAGCGCTCACAAT-3′.
This single-stranded DNA contains an 18 nucleotide analogue of the natural lac operator sequence, (the underlined portion), described in Simons et al., 1984, Proc. Natl. Acad. Sci. USA, vol. 81, pp. 1624-1628, and Sadler et al., 1983, Proc. Natl. Acad. Sci. USA, vol. 80, pp. 6785-6789, and was self-annealed to form the following double-stranded structure:

```
5'-TCGAATTGTGAGCGCTCACAAT      -3'
3'-     TAACACTCGCGAGTGTTAAGCT-5'
        * * * * * * * * * * * * * * * * * * * * *
```

Thus, the full 22 bp palindromic operator sequence (shown above underlined by asterisks), described by Simons et al., supra, was created during this step. This DNA has XhoI-compatible sticky ends and was cloned into the unique XhoI site of pX-8, described in Fromm et al., 1982, J. Mol. Appl. Genet., vol 1, pp. 457-481. The resulting plasmid (pSVlacO) was digested with HindIII, and the 1.1 kb promoter/operator containing fragment was ligated to the unique HindIII site of pSVOCAT, described in Gorman et al., 1982, Mol. Cell. Biol., vol. 2, 1044-1051, to generate pSVlacOCAT, described in Brown et al., 1987, Cell, vol. 49, pp. 603-612.

Plasmid pHSVNEO can be constructed using readily available materials by linking a thymidine kinase promoter (tk) derived from herpes simplex virus ("HSV") to a neomycin resistance gene ("NEO"), and then cloning this chimeric sequence in pBR322.

Transfection and Selection of Stable Transfected Mammalian Cell Lines.

CV-1P and its G418-resistant derivative cell lines ("ClOCAT") were grown on plastic surfaces (Becton-Dickinson) in Dulbecco's modification of Eagle's minimal essential medium ("DME", Gibco) supplemented with 10% newborn calf serum ("NCS", Flow Laboratories, Rockville, MD). The antibiotic G418 (Gibco) was added to the medium as indicated. Cells were cultivated at 37°C in a humidified atmosphere containing 10% $CO_2$. Isopropyl β-D-thiogalactoside ("IPTG", Bethesda Research Laboratories) was prepared as a 1 M stock solution in 1 x PBS (0.01 M sodium phosphate [pH 7.4], 0.14 M NaCl); and aliquots were added to the indicated cultures, as needed.

Supercoiled plasmid DNA was introduced into CV-1P cells by a calcium phosphate coprecipitation technique generally as described by Graham et al., 1973, Virology, vol. 52, pp. 456-467, followed 4 hours later by a glycerol shock, generally as described in Parker et al. 1979, J. Virol., vol. 31, pp. 360-369, and in Wigler et al., 1979, Proc. Natl. Acad. Sci. USA, vol. 76, pp. 1373-1376. Specifically, 1.5 ml of DNA/CaCl₂ solution containing 42 μg of pCMVlacI, 3 μg of pSVlacOCAT, and 3 μg of pHSVNEO was added dropwise to 1.5 ml of twice-concentrated HEPES-buffered saline ("2 x HBS" [pH 7.05]). This solution was mixed by bubbling with air and was allowed to precipitate for 15-20 min. One ml of precipitate was subsequently added to each of three 80% confluent 60 mm plates of CV-1P cells. After four hours, cells were drained of medium and then exposed to 15% glycerol in 1 x HBS (pH 7.05) for three minutes at 37°C. Three days after transfection, cells were trypsinized and transferred to 100 mm plates containing DME supplemented with 10% NCS and G418 (400 μg/ml), as described by Southern et al., 1982, supra. Individual G418-resistant colonies were trypsinized in cloning cylinders and transferred to plastic microtiter wells. Clonal lines were expanded and maintained in DME containing 10% NCS and G418 (100 μg/ml).

Stable lacI⁺ cell lines containing an integrated chloramphenicol acetyl transferase ("CAT") gene under control of a hybrid SV40 promoter bearing a lac operator were produced as follows. Monkey kidney cells (CV-1P) were cotransfected with a plasmid encoding lac repressor (pCMVlacI, Fig. 1), a plasmid bearing a CAT coding sequence linked to the operator-containing SV40 promoter element (pSVlacOCAT, Fig. 2), and a plasmid containing the neomycin (NEO) resistance gene (pHSVNEO), in a molar ratio of 16:1.0:1.2. Clones were selected in medium supplemented with G418, and all 43 clones that developed into stable G418-resistant cell lines were analyzed. Identical replicate cultures of actively growing cells from each line were cultivated in parallel with medium containing (+) or lacking (-) 15 mM IPTG for 3 to 4 days. At the end of the incubation period, the cells had reached confluence and were then harvested for preparation of extracts. Paired sets (+, -) of extracts from each cell line were assayed, in parallel, for protein content and CAT activity. Aliquots of extracts derived from three IPTG-treated lines (ClOCAT lines A4, A8, and A11) each showed CAT activity. In the absence of IPTG, the level of CAT activity in the paired, untreated cultures was similar to that of parental CV-1P cells (see below). A fourth line (ClOCAT line A22) showed constitutive expression of CAT activity in the absence or presence of IPTG. The remaining 39 lines revealed background CAT activity in the presence or absence of IPTG.

A quantitative Western blot analysis performed on matched extracts from the three clonal G418-resistant lines exhibiting an inducible CAT⁺ phenotype and from parental CV-1P cells showed that these transformed cell lines were synthesizing intact lac repressor.

These results demonstrate that IPTG can regulate the CAT phenotype of these mammalian cells, strongly implying that CAT coding unit(s) are stably present within the cells. To establish that the CAT coding unit(s) were integrated into high molecular weight DNA, one of the IPTG-inducible lines was studied after it had been passaged in culture for 6 months. Specifically, restriction digest products of cell DNA from ClOCAT A8 were analyzed by agarose gel electrophoresis. A blot of the gel was incubated with a 1.6 kb probe containing the CAT coding sequence and the SV40 early splicing and polyadenylation signals. HindIII cleaves pSVlacOCAT plasmid DNA (5.6 kb) at two sites to yield a CAT-containing fragment of 4.5 kb. In the HindIII digestion of A8 DNA, a single dominant band at 7.5 kb was detected along with several much less intense faster migrating bands. Digestion of A8 DNA with enzymes known to be noncutters of pSVlacOCAT, including XbaI and XhoI only resulted in the appearance of high molecular weight hybridizing fragments (≧ 9.4 kb). A double digest with HindIII and Xba reduced the size of the dominant HindIII band from 7.5 kb to 6.0 kb, leaving only a minor 7.5 kb band that is likely a residual 7.5 kb HindIII band. Restriction digests of CV-1P DNA with HindIII, EcoRI, XbaI, and XhoI yielded no hybridizing bands. These results are most consistent with integration of a single unit of pSVlacOCAT into high molecular weight DNA in the configuration shown in Fig. 4. Since these cells were actively growing in culture for six months prior to this analysis, these results show that the CAT coding unit is integrated into a chromosome of the host cell. An absence of bands smaller than 23 kb in uncut A8 DNA reflects the absence of measurable quantities of free plasmid DNA in this line.

Induction of CAT Expression by IPTG.

Two demonstrations were designed to measure the rate of appearance and disappearance of CAT activity following addition and removal of IPTG from cultures of inducible ClOCAT lines, to evaluate the kinetics of induction in mammalian cells. First, plates were seeded, in parallel, with cells from stock cultures of CV-1P and ClOCAT A4, A8, and A11 at densities which allowed cells in the relevant plates to grow for 48, 72, or 96 hours before reaching confluence. IPTG (15 mM) was present in the media bathing these actively growing cells throughout the indicated time intervals. Control plates containing growing cells from each line were incubated in IPTG-free medium. At the end of each designated time period, extracts were prepared from all cell lines and assayed, in parallel, for protein content as described generally in M.M. Bradford, 1976, Anal. Biochem., vol. 72, pp. 248-54. Then, aliquots of each cell extract containing 445 µg of protein were adjusted to an equivalent final volume (150 µl) and assayed, in parallel, for CAT activity. In the inducible ClOCAT lines, CAT activity increased in a nearly linear fashion over a 3 to 4 day period in the presence of 15 mM IPTG, while CAT activity in extracts from CV-1P cells and from control ClOCAT cells not exposed to IPTG remained at background levels. Maximum CAT activity was reached by 4 days certainly in one case, and most probably in the other cases, since incubation, and passage of these cells for periods of up to 12 days in the presence of 15 mM IPTG resulted in no further increase in CAT activity. In lacI+ E. coli, the inducing agent isopropyl β-D-thiogalactoside (IPTG) is capable of activating expression of β-galactosidase within several minutes. Thus, the induction kinetics in these cells are markedly different from the induction kinetics in E. coli. The mean basal percent acetylated chloramphenicol per 445 µg of protein (± S.E.M.) as measured in the ClOCAT cell extracts was only slightly higher than that detected in the parental, CV-1P cell extracts. In other runs there was no significant difference between these values. Taking the mean level of CAT activity in CV-1P cells as a background level, the mean specific CAT activity increased, at a minimum, 32 fold in the ClOCAT cells by incubation with 15 mM IPTG for 4 days. All three of the independently derived clonal lines displayed similar behavior, when assayed under identical conditions.

A second demonstration was showed that the kinetics of re-repression after IPTG removal followed a similar course. Parallel plates of actively growing cells from CV-1P, and ClOCAT lines A4, A8, and A11 were incubated for 96 hours in medium containing 15 mM IPTG. At the end of this period, half of the cells from each line were extracted, and the remainder were subcultured at densities which allowed their active growth for an additional 24, 48, or 72 hours in the absence of IPTG. As negative controls, replicate plates of actively growing cells from all these lines where cultured in the absence of IPTG. Cells were harvested at the indicated time intervals, and all extracts were assayed in parallel for protein content. Aliquots of each cell extract containing an identical quantity of protein (445 µg) were adjusted to an equivalent final volume and assayed, in parallel, for CAT activity. By three days after IPTG removal, steady state CAT activity in all three IPTG-treated ClOCAT clonal lines had decreased from its value at the 96 hour time point to background levels. In contrast, CAT activity from extracts of CV-1P cells as well as from the replicate plates of ClOCAT cells which were never exposed to IPTG remained at background levels. Thus, the kinetics of induction and re-repression of CAT gene expression appear to follow a similar, slow time course in these monkey cells. Moreover, following removal of the inducer, apparently complete re-repression of the CAT gene was detected.

The functional dependence of induction of CAT activity on IPTG concentration was demonstrated as follows. Replicate plates of ClOCAT cells were actively grown for 4 days in medium containing various concentrations of IPTG. In three separate experiments of this type, the concentration of IPTG was varied through a low-dose (1-500 µM), a medium-does (0.5-15 mM), and a high-dose interval (5-50 mM). Cells were harvested after 4 days, and extracts were assayed for protein content and CAT activity, as in previous experiments, except that CAT assays were run for 90 minutes. At lower IPTG concentrations CAT activity increased in line A11 as a function of the logarithm of the IPTG concentration beginning at 25 µM. The other 2 lines showed similar responses. CAT activity increased as a linear function of IPTG concentration in all three lines when tested over the medium dose range. At higher doses of IPTG, CAT activity in line A11 also increased continually over the entire IPTG range. The other two lines were not tested in this manner. At IPTG concentrations greater than 50 mM, toxicity was observed, as reflected by a reduction in growth rate and/or plating efficiency. Specific CAT activity as measured by percent acetylated chloramphenicol per 445 µg of protein increased 60 fold in two replicate assays after incubation of A11 cells with 50 mM IPTG for four days.

The following demonstrates that growth arrested cells can be induced by IPTG. Plates of confluent cells (A4 and A11) were cultivated for four days in the presence or absence of 15 mM IPTG. Parallel plates of confluent cells were grown and counted for cell number on day 1 and day 4. After the four day incubation period, cell extracts were prepared as previously described and assayed for protein content and CAT activity. Significant IPTG-mediated induction of CAT activity occurred in these contact-inhibited growth-arrested cells by comparison with parental CV-1P cells. To verify that the cells were growth arrested, the results of cell counts for line A11 in the presence of IPTG showed an average of 6.6 x $10^6$ cells per plate on day 1 and 6.8 x $10^6$ cells per plate on day 4. In the absence of IPTG there were an average of 4.8 x $10^6$ cells per plate on both days 1 and 4. Thus, the induction of CAT activity by IPTG does not require active cell division.

## Other Embodiments

Other embodiments are within the following claims. For example, other functional repressors and functional operators derived from the E. coli lac repressor-operator system can be used. For example, other mutant or synthetic analogues of the E. coli lac operator can be used. A synthetic palindromic operator such as that used in the example described above, see A. Simons, supra, is preferred because although it binds with a high affinity specifically to lac repressor, it can, when so bound, be induced to disassociate by an allolactose analog such as IPTG.

Other analogs of allolactose than IPTG can be used as inducers to bind the lac repressor and thereby to reduce the binding affinity of the repressor for the operator, activating transcription of a gene linked to the operator, see M.D. Barkley et al., supra. IPTG is preferred because lac repressor bound to IPTG has a 300-fold lower affinity for lac operator, and because when lac repressor is bound to a preferred high affinity lac operator, IPTG induces disassociation of the repressor from the operator.

Prokaryotic repressors and their cognate operators derived from operon systems other than the E. coli lac operon can be used. For example, a gal repressor and its cognate gal operator, derived from the galactose operon of E. coli, can be used according to the invention to regulate expression of a gene, linked to the gal operon, integrated into a chromosome of a mammalian cell. Galactose or a gal repressor-binding analog can be used as an inducer. And, for example, a trp aporepressor and its cognate trp operator, derived from the tryptophan operon of E. coli, can be used according to the invention to regulate expression of a gene, linked to the trp operator, integrated into a chromosome of a mammalian cell. Transcription of the trp operator-linked gene can be inhibited by the presence of tryptophane, which binds the aporepressor to inhibit operator-binding by the trp repressor.

## Claims

1. A method for achieving expression of a desired protein in a mammalian cell, comprising
transfecting said mammalian cell with a eukaryotic promoter coupled to a DNA sequence encoding a prokaryotic repressor,
transfecting said mammalian cell with a prokaryotic operator linked to a DNA sequence encoding said desired protein and a promoter driving the transcription of said linked DNA sequence, said operator and said linked DNA sequence being integrated into a chromosome of said cell, said operator and said linked DNA sequence and said promoter and said repressor being functional in said cell, and
contacting said cell with a substance that binds said repressor.

2. The method claimed in claim 1 in which said repressor and said operator are both lac or both gal, or wherein said repressor is a trp aporepressor and said operator is a trp operator.

3. The method claimed in claim 2 in which said substance binding said lac repressor is allolactose or an analog thereof.

4. The method claimed in claim 3 in which said analog is isopropyl-β-D-thiogalactoside.

5. The method claimed in claim 2 in which said substance binding said gal repressor is galactose or an analog thereof.

6. The method claimed in claim 2 in which said substance binding said trp aporepressor is tryptophane or an analog thereof.

7. A mammalian cell comprising a DNA sequence encoding a functional prokaryotic repressor and a functional cognate operator, said operator being integrated into a chromosome of said cell.

8. The cell of claim 7, further comprising, linked to said operator, a DNA sequence encoding a desired protein and a promoter driving the transcription of said linked DNA sequence.

9. A non-human mammalian organism comprising a cell claimed in claim 7 or 8.

10. A method for regulating production of a desired protein in a non-human mammal which comprises providing said mammal with cells having integrated into a chromosome thereof a functional DNA sequence encoding a prokaryotic operator linked to a DNA sequence encoding said desired protein and a promoter driving the transcription of said linked DNA sequence,
said cell also having a functional DNA sequence encoding a prokaryotic repressor coupled to a eukaryotic promoter,
and allowing said cells to grow either in the presence or in the absence of a substance binding to said repressor.

## FIG.1

pUC 18

Ssp I

Nde I
Nar I
Pvu II
Sal I
Nde I
Nar I

CMV IE94
promoter
segment

pCMVlacI
5.5 Kb

AMP

ORI

ATG

EcoRI
Mae III

Mlu I

Mae III
Apa I

EcoRV

lacI

Pvu II
EcoRI
Pvu II
Nar I
Pvu II

## FIG.2

pSVlacOCAT
5.6 Kb

BamHI

Hind III

PO

CAT

Hind III

## FIG.3

Hind III
PO
BamHI
CAT
XbaI
Hind III

probe